# EUROPEAN PATENT SPECIFICATION

(11) **EP 2 940 023 B1**
(45) Date of publication and mention of the grant of the patent: **15.11.2017**
(21) Application number: 14184064.5
(22) Date of filing: 09.09.2014
(51) Int. Cl.: C07D 493/04, C08K 5/1575

(54) **METHOD OF PREPARING DIACETAL-BASED CLARIFYING AGENT**
VERFAHREN ZUR HERSTELLUNG EINES DIACETALBASIERTEN KLÄRMITTELS
PROCÉDÉ DE PRÉPARATION D'UN AGENT DE CLARIFICATION À BASE DIACÉTALE

(30) Priority: 30.04.2014 TW 103115454
(43) Date of publication of application: 04.11.2015
(73) Proprietor: SUNKO INK CO., LTD., Dali Dist. Taichung City (TW)
(72) Inventor: Huang, Ting-Ti, Pingzhen City, Taoyuan County (TW); Tsou, Chiu-Peng, Pingzhen City, Taoyuan County (TW); Wei, Wen-Chen, Pingzhen City, Taoyuan County (TW)
(74) Representative: Geurts, Franciscus Antonius

(56) References cited:
- EP-A1- 0 569 198
- EP-A1- 1 505 109
- US-A- 5 023 354

## Description

### 1. Field of the Invention

The present invention relates to a method of preparing a clarifying agent for polyolefin, more particularly to a method of preparing a diacetal-based clarifying agent.

### 2. Description of the Prior Arts

Transparent plastics enable visibility of contents thereof and thereby provide users with significant convenience, which thus allow the broad application of transparent plastics in the fields of household utensils, electronic appliances, medical apparatuses, automobile anti-freezing fluid containers, and food packaging. The widespread use of transparent plastics then demands supply of transparent plastic materials and therefore has made clarifying additives a crucial issue for the research and development of transparent plastics. One of the most well-known clarifying agents is the diacetal-based clarifying agent.

Diacetals added to polymers, such as polyolefin, act as a nucleating agent that facilitates crystallization. The facilitated crystallization shortens the time for molding during a melting process, which also contributes to the improvement of physical properties of the plastics being processed. In addition, diacetals, as a clarifying agent, raise the transparency of semi-crystalline polymers.

A commonly accepted understanding on the mechanism of a diacetal-initiated clarification process is as follows. Diacetal powders added to a polyolefin plastic and melted at a suitable temperature afterwards crystallize and disperse in the cooling and molding process of the polyolefin plastic. The crystallized and dispersed diacetal develops a crystallizing network. In the crystallizing network, a multitude of spherical nucleation sites are formed. The nucleation sites are of a size too small to initiate scattering of visible light, thereby allowing the transparency of the molded polyolefin plastic product as a transparent final product. Therefore, miniaturization of diacetal nucleation sites, prevention of aggregation of diacetal products, and increase of purity of diacetal are key factors to improve the optical properties of polyolefin plastics.

With reference to Japanese Pat. Appl. No. 94424/1974, diacetal-based clarifying agents, such as dibenzylidene sorbitol (DBS), originated from aromatic benzene aldehydes and polyols, are commonly used to improve the transparency of plastic product. U.S. Pat. No. 4016118 (Hamada et al.*,* E.C. Chemical Industries & Co., Ltd.; Itoh & Co., Ltd.) discloses a polyolefin composition comprising 0.1% to 0.7% of DBS with improved transparency and reduced molding shrinkage. Other DBS derivatives, such as 1,3:2,4-di(4-methylbenzylidene)-D-sorbitol (MDBS), 1,3:2,4-di(4-chlorobenzylidene)-D-sorbitol (CDBS), 1,3:2,4-di(4-ethylbenzylidene)-D-sorbitol (EDBS) and 1,3:2,4-di(3,4-dimethylbenzylidene)-D-sorbitol (DMDBS), are developed as commercially available clarifying agents for polyolefin.

As demonstrated in U.S. Pat. No. 5574174 (Syed, Montell North America Inc.), diacetal-based clarifying agents, such as asymmetric 1,3-di(substituted arylidene)-2,4-di(substituted thenylidene)-D-sorbitol, which are synthesized by aromatic thioheterocyclic aldehydes and polyols, are also available. Taiwan Pat. No. 307340 (Tsou et al., Kuo Ching Chemical Co., Ltd.) discloses symmetric di(substituted thenylidene)-D-sorbitol, which can be applied to polyolefin for improving transparency.

U.S. Pat. No. 4429140 (Murai et al., New Japan Chemical Co., Ltd.) discloses a method of preparing DBS by mixing benzaldehyde or alkyl acetal derivatives with sorbitol in the presence of an acid catalyst, hydrophobic organic solvent and water-soluble polar organic solvent to undergo a condensation reaction.

However, diacetal prepared by the foregoing conventional methods generally incurs undesirable by-products and residues of impurities and intermediates, such as reducing sugar, aromatic aldehyde, aromatic acid, acid catalysts and alkyl acetal derivatives. During plastic thermal processing, a diacetal composition comprising these thermal unstable impurities or intermediates is decomposed into aromatic aldehydes and substances having low boiling point, thereby releasing stinking odor, reducing the final plastic product's transparency, and degrading the final plastic product's property and appearance. When applying the final plastic product to food package, the stinking odor released therefrom can also be transferred to the content, *e.g*. food, wrapped in the final plastic product, thereby limiting the final plastic product's application.

To overcome the foregoing drawbacks, there have been efforts on modifying the preparation of diacetal and improving the purity thereof. Taiwan Pat. No. 565562 (Scrivens et al., Milliken & Company) discloses a method of preparing DBS with a purity about 90% to 97% including the condensation of aromatic aldehyde and polyol having five or more hydroxyl groups in the presence of an acid catalyst, a hydrophobic organic liquid medium, and an additive selected from dihydric, trihydric and tetrahydric alcohols and then purification to obtain DBS. However, the method as taught by Scrivens *et al.* still fails to inhibit the release of stinking odor and the occurrence of yellowing under thermal treatment.

U.S. Pat. No. 6518339 (Sheppard et al., Milliken & Company) discloses a method of introducing an amine, such as hydrazine or hydrazide, in DBS-based clarifying agent to reduce the aldehyde residue in the clarifying agent. However, the amine employed causes yellowing; in addition, the hypertoxic hydrazine and hydrazide remaining therein also lead to less guaranteed safety in the use of final plastic product. Therefore, the method still cannot obviate the limitations in various uses.

Taiwan Pat. No. I318994 (Ishikawa et al., New Japan Chemical Co., Ltd.) discloses an agent comprising C₆ to C₃₂ saturated or unsaturated aliphatic carboxylic acid, an anionic surfactant and at least one aliphatic amine for suppressing the transferring of the stinking odor released from DBS. Nevertheless, Ishikawa *et al.* does not mention the removal of the suppressed impurities and the suppression effects towards the odor. Ishikawa *et al.* also discloses a lipophilic suppression composition comprising said agent in conjunction with the polymeric material. If the lipophilic suppression composition remains in the final plastic product, the lipophilic suppression composition may be transferred into the oil food, and limit its application in food packaging.

U.S. Pat. No. 7897663 (Tsou et al., Kuo Ching Chemical Co., Ltd.) discloses a clarifying agent composition comprising dithenylidene sorbitol and DBS. Further, Tsou *et al.* also discloses a method of condensing aromatic aldehyde and polyol in the presence of an acid catalyst, polar organic solvent and then adding an organosilane treated fume silica having a pH value of 5.5 to 8 to the mixture to control the size of the produced diacetal powders. By using the diacetal powders as a clarifying agent, Tsou *et al.* does improve the dispersion characteristic of the diacetal powders and produce a plastic product without white spots. Tsou *et al*., however, fails to reduce or inhibit the generation of the stinking odor or yellowing at high temperature processing.

Accordingly, there is a need to improve the method of preparing a diacetal-based clarifying agent for inhibiting the release of the intolerable odor during the plastic processing and yellowing under thermal treatment, so as to improve the color stability of the final plastic product, meet the safety requirements, and expand the final plastic product's applications.

An objective of the present invention is to allow the impurities with stinking odor contained in the conventional clarifying agent to be reacted into the substances with low odor and easy to be purified, for inhibiting the release of intolerable odor during the thermal treatment of the conventional clarifying agent in the plastic process.

Another objective of the present invention is to remove the odor from the clarifying agent without using toxic agent, so as to improve the safety and the applications of the diacetal-based clarifying agent and the final plastic comprising the same, thereby meeting the safety requirements.

Further another objective of the present invention is to increase the color and thermal stabilities for expanding the final plastic product's applications.

To achieve the aforementioned objectives, the present invention provides a method of preparing a diacetal-based clarifying agent, comprising the steps of: (a) mixing an aromatic aldehyde, a polyol, and an acid catalyst in a polar organic solvent to obtain a first reaction mixture; (b) adding a hydrogenating agent into the first reaction mixture to obtain a precipitated product; (c) mixing methanol and the precipitated product to obtain a second reaction mixture; (d) filtering the second reaction mixture to obtain a solid substance; and (e) drying and grinding the solid substance to obtain the diacetal-based clarifying agent, wherein the diacetal-based clarifying agent is a compound selected from the group of compounds represented by formulae (I) to (V) as described below, and wherein an equivalence ratio of said aromatic aldehyde to said polyol ranges from 2:1 to 2:2 and an equivalence ratio of the hydrogenating agent to aromatic aldehyde is more than 0.01:1.

Preferably, the equivalence ratio of the hydrogenating agent to aromatic aldehyde is within 0.03:1 and 0.3:1.

Preferably, the equivalence ratio of aromatic aldehyde to said polyol ranges from 2:1.05 to 2:1.3.

Preferably, the hydrogenating agent applicable to react with said aromatic aldehyde may be sodium hydride (NaH), potassium hydride (KH), aluminium hydride (AlH), sodium cyanoborohydride (NaBH₃(CN)), diisobutylaluminium hydride ((i-C₄H₉)₂AlH), lithium borohydride (LiBH₄), sodium borohydride (NaBH₄), potassium borohydride (KBH₄), calcium borohydride (Ca(BH₄)₂) or combinations thereof, for assuring the process safety of diacetal-based clarifying agent. More preferably, the hydrogenating agent is NaBH₄ or KBH₄.

Said hydrogenating agent may be made in powder, tablet or liquid form. The commercially available hydrogenating agent comprises, but is not limited to: VenPure™ AF (highly pure sodium borohydride tablets), VenPure™ SF (highly pure sodium borohydride powders), VenPure™ (solution including sodium hydroxide and sodium borohydride, concentration adjustable) or VenPure K™ (highly pure potassium borohydride powders).

Preferably, the aromatic aldehyde applicable to the present invention may be a thiophenecarboxaldehyde-based compound, a benzaldehyde-based compound or their combination. Said thiophenecarboxaldehyde-based compound may be an unsubstituted thiophenecarboxaldehyde or a substituted thiophenecarboxaldehyde having 1 to 3 functional group(s), the functional group is selected from the group consisted of: an alkyl group having 1 to 4 carbon atoms, an alkoxy group having 1 to 4 carbon atoms, an alkoxycarbonyl group having 1 to 4 carbon atoms, and a halo group such as fluoro group, chloro group, or bromo group. Specifically, said thiophenecarboxaldehyde-based compound may be 3-thiophenecarboxaldehyde or 5-methyl-2-thiophenecarboxaldehyde. Said benzaldehyde-based compound may be an unsubstituted benzaldehyde or a substituted benzaldehyde having 1 to 3 functional group(s), the functional group is selected from the group consisted of: an alkyl group having 1 to 4 carbon atoms, an alkoxy group having 1 to 4 carbon atoms, an alkoxycarbonyl group having 1 to 4 carbon atoms, and a halo group such as fluoro group, chloro group, or bromo group. Specifically, said benzaldehyde-based compound is 4-methyl-benzaldehyde, 4-ethyl-benzaldehyde or 3,4-dimethyl-benzaldehyde.

Preferably, the polyol applicable to the present invention may be D-sorbitol or D-xylitol. More preferably, the polyol is D-sorbitol.

Preferably, the acid catalyst may be sulfuric acid, phosphoric acid, hydrochloric acid, methanesulfonic acid, camphor sulfonic acid, *p*-toluene sulfonic acid or naphthalene sulfonic acid or their derivatives. More preferably, the acid catalyst is methanesulfonic acid or camphor sulfonic acid.

Preferably, the polar organic solvent may be hydrophilic solvent such as methanol, ethanol, dimethyl formamide, acetonitrile, water or combinations thereof. Said solution used for mixing with the precipitated product is methanol.

In accordance with the method of the present invention, the undesired reactants, intermediates and the impurities can be dissolved in the solution and diacetals can be precipitated under a mild reaction condition, such that the highly pure final products, i.e., diacetal-based clarifying agent, can be easily removed from the reactants, intermediates and the impurities by filtering and washing steps without dangerous and toxic steps. Therefore, the method of preparing the diacetal-based clarifying agent is much simpler and safer than the conventional method.

By adopting an appropriate hydrogenating agent reacting with the first reaction mixture, those undesired materials including reactants, intermediates, and the impurities in the first reaction mixture, which have low thermal stability and apt to release stinking odor, can be reacted into low odor and easily-purified substances. Accordingly, the diacetal-based clarifying agent free of impurities and without stinking odor can be easily prepared by merely dissolving the precipitated product in a specific polar organic solvent, filtering and drying the mixture.

The condensation reaction between aromatic aldehyde and polyol can be conducted at different temperatures depending on the materials of aromatic aldehyde, polyol and acid catalyst. For example, 3-thiophenecarboxaldehyde can be reacted with the polyol in the presence of acid catalyst and polar organic solvent at room temperature to perform a condensation reaction.

Preferably, the step of "adding a hydrogenating agent into the first reaction mixture with an equivalence ratio of the hydrogenating agent to aromatic aldehyde more than 0.01:1 to obtain a precipitated product" comprises: (b1) adding the hydrogenating agent into the first reaction mixture with the equivalence ratio of the hydrogenating agent to aromatic aldehyde more than 0.01:1; and (b2) adjusting the pH value of the first reaction mixture within a range from pH 7 to pH 13, so as to obtain the precipitated product.

Preferably, said first reaction mixture can be adjusted as a basic solution within the range from pH 8 to pH 11, for inhibiting the reverse reaction and reducing the production of the impurities with odor, such as aldehyde, and facilitating the removal of the acid catalyst from the first reaction mixture. The stability of the diacetal-based clarifying agent can be improved by purifying the crude product under a basic condition.

The diacetal-based clarifying agent prepared by the method of the present invention is a compound selected from the group consisting of compounds represented by formulae (I) to (V): 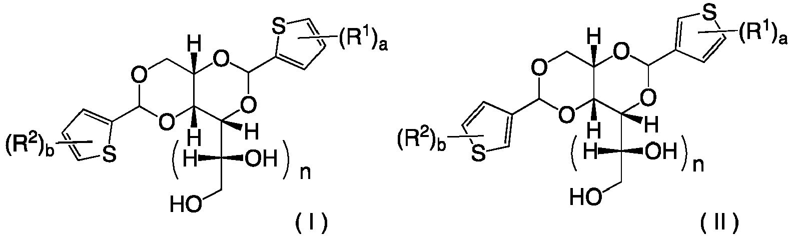 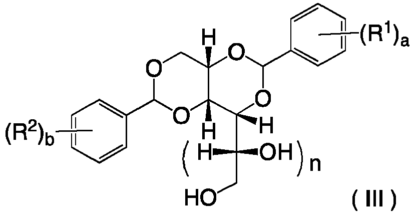 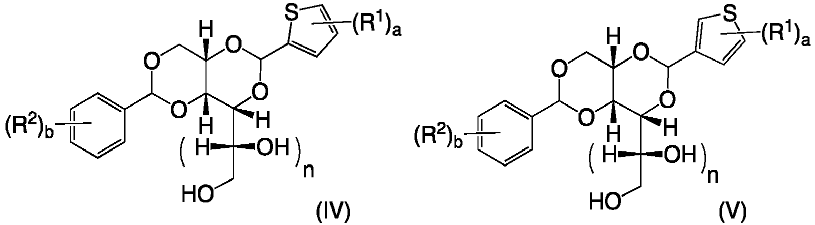 wherein R¹ and R² are each independently selected from the group consisting of a hydrogen group, an alkyl group having 1 to 4 carbon atoms, an alkoxy group having 1 to 4 carbon atoms, an alkoxycarbonyl group having 1 to 4 carbon atoms, a fluoro group, a chloro group, or a bromo group; a and b are each independently an integral from 0 to 3; and n is 0 or 1.

Preferably, the purity of the obtained diacetal-based clarifying agent is more than 97%, and the residue of the aromatic aldehyde, as impurities, is less than 50 ppm. More preferably, the purity of the diacetal-based clarifying agent is more than 99%.

In another embodiment of the present invention, the diacetal-based clarifying agent prepared from the method may further comprises organosilane treated fume silica powders dispersed between the diacetal powders. The organosilane treated fume silica powders have a pH value of 5.5 to 8, and the amount of the organosilane treated fume silica powders is from 2 wt% to 50 wt% based on the total amount of the diacetal-based clarifying agent.

Herein, the organosilane treated fume silica powders having a pH value of 5.5 to 8 is measured in a 4% w/w dispersion in 1:1 mixture of water-methanol.

Preferably, the material of the organosilane treated fume silica powders is selected from fume silica powders treated with dimethylsilicon fluid, polydimethylsiloxane, or hexamethyldisilazane. More specifically, the commercially available organosilane treated fume silica powders comprises: H-2000 by Wacker Chemicals East Asia Limited (hexamethyldisilazane treated fume silica, pH 6.7 to 7.7, surface area 140±30 m²/g), TS-720 by Cabot Corporation (dimethylsilicon fluid and polydimethylsiloxane treated fume silica, pH value 5.8, surface area 105 to 130 m²/g), and TS-530 by Cabot Corporation (hexamethyldisilazane treated fume silica, pH 6.0, surface area 205 to 245 m²/g).

More specifically, the diacetal-based clarifying agent mainly comprises the diacetal compound selected from the group consisted of: 1,3:2,4-di(3-thenylidene)-D-sorbitol, 1,3:2,4-di(5-methyl-2-thenylidene)-D-sorbitol, 1,3:2,4-di(4-methyl-benzylidene)-D-sorbitol, 1,3:2,4-di(3,4-dimethyl-benzylidene)-D-sorbitol, 1,3-(3-thenylidene)-2,4-(4-methyl-benzylidene)-D-sorbitol, 1,3-(3-thenylidene)-2,4-(3,4-dimethyl- benzylidene)-D-sorbitol, 1,3-(5-methyl-2-thenylidene)-2,4-(4-methyl- benzylidene)-D-sorbitol, or 1,3-(5-methyl-2-thenylidene)-2,4-(3,4-dimethyl- benzylidene)-D-sorbitol.

The present invention also relates to a plastic composition without odor, comprising the highly pure diacetal-based clarifying agent as mentioned above and polyolefin.

Preferably, the amount of the highly pure diacetal-based clarifying agent ranges from 0.005 wt% to 2 wt% based on the total amount of the plastic composition. More preferably, the amount of the highly pure diacetal-based clarifying agent ranges from 0.05 wt% to 0.5 wt%, and further more preferably, the amount of the highly pure diacetal-based clarifying agent ranges from 0.1 wt% to 0.3 wt% based on the total amount of the plastic composition.

Preferably, the polyolefin material comprises crystalline and semi-crystalline polyolefin polymers or polyolefin resin. Said polyolefin material mainly comprises at least one homopolymer of an aliphatic mono-olefin or a copolymer of an alpha-mono-olefin having 2 to 8 carbon atoms, particularly propylene and mixture of polyolefin.

The term "semi-crystalline" used herein is directed to the crystallinity of at least about 5% to 10% as measured by X-ray diffraction.

With the technical means of addition of hydrogenating agent into the first reaction mixture and use of the applicable polar organic solvent, the method in accordance with the present invention can remove the stinking odor and thermally unstable impurities, including reducing sugar, aromatic aldehyde, aromatic acid, acid catalysts and alkyl acetal derivatives from the crude product, thereby inhibiting the release of the intolerable odor during the plastic processing effectively.

The diacetal-based clarifying agent prepared by the method of the present invention is substantially free of any impurities with stinking odor and the hydrogenating agent and polar organic solvent used in the method are not hypertoxic, such that the safety and applications of the diacetal-based clarifying agent and the final plastic product can be further improved.

Since the diacetal-based clarifying agent prepared by the method of the present invention can have high purity and good thermal stability at high temperature, applying the diacetal-based clarifying agent to the plastic process not only avoids yellowing of a final plastic product but also provides a better color stability than that of the commercial product, and thereby improving the final plastic product's industrial application and applicability.

Other objectives, advantages and novel features of the invention will become more apparent from the following detailed description when taken in conjunction with the accompanying drawings.

Hereinafter, one skilled in the arts can easily realize the advantages and effects of the present invention from the following examples. Therefore, it should be understood that the descriptions proposed herein are just preferable examples for the purpose of illustrations only, not intended to limit the scope of the invention. Various modifications and variations could be made in order to practice or apply the present invention without departing from the spirit and scope of the invention.

The methods and conditions used to analyze the diacetal-based clarifying agent obtained in the following Examples and Comparative Examples are described as follows.

### 1. Yield

The theoretical mass of diacetal-based compound was determined based on aromatic aldehyde as limiting agents. The yield values were calculated from dividing the actual mass by the theoretical mass and then multiplying 100%.

### 2. Melting point

The melting point analyses were carried out with a BUCHI melting point instrument B-540. The sample powders were placed into a capillary tube for melting point analyses around 1 centimeter in height. Then the capillary tubes filled with the sample powders were inserted in the slot of the instrument. During the experiments, the sample powders were heated rapidly up to 200°C for a steady state, and then heated with a heating rate of 2°C per minute, in order to observe and record the temperature while the white powders started melting and fully melted.

### 3. Residue of aromatic aldehyde

A SHIMADZU gas chromatography GC-2014 equipped with a J&W column HP-1 was used herein. The column had a length of 30 meters, an internal diameter of 0.32 millimeters, and a film thickness of 0.25 micrometers. The injection temperature was set at 200°C, and the detection temperature was set at 280°C. The column ran with helium gas as a carrier gas with a flow rate of 2 mL per minute. The split ratio was 10:1. During the experiment, the column temperature was set at 100°C for 3 minutes in the initial state, then heated with a heating rate of 15°C per minute, and finally kept at 230°C for 9 minutes.

The retention times of different aromatic aldehydes were: 5-methyl-2-thiophenecarboxaldehyde (5.23 minutes); 3-thiophenecarboxaldehyde (3.69 minutes); 4-methyl-benzaldehyde (4.83 minutes); 4-methyl-benzaldehyde (6.04 minutes); and 3,4-dimethyl-benzaldehyde (6.51 minutes). Said retention time of the aromatic aldehydes varied and shifted slightly depending on different gas chromatography instruments and columns with different theoretical plate numbers.

In the experiment, 1 gram of different diacetal sample powders, collected from the following Examples and Comparative Examples, was dissolved in 10 mL of methanol, and then ultrasonicated for 30 minutes to obtain a mixture. After filtering the mixture, the filtrates were collected to determine the amounts of aromatic aldehydes. If the absorption peaks of the aromatic aldehydes were not observed, the results were represented by "not detected". Herein, the instrument detection limit (IDL) was 1 ppm, and the detection limit of the sample was 10 ppm.

### 4. Purity of diaetal compound

A Waters 2487 liquid chromatography (LC) equipped with a LiChrospher® 100 column RP-18 was used herein. The column had a length of 25 meters, an internal diameter of 4 millimeters, a film thickness of 5 micrometers, and an outer temperature of 30°C. The elution solvent used was a mixture of acetonitrile and water (v/v 65/35) of HPLC-grade. The sample powders were dissolved in dimethyl formamide and then diluted with methanol. The concentration of the samples were 1000 ppm, and each injection amount was 20 microliters.

The wavelengths of UV radiations and retention times of sample powders (different diacetal compounds) were:
1,3:2,4-di(5-methyl-2-thenylidene)-D-sorbitol (254 nm, 6.96 minutes);
1,3:2,4-di(3-thenylidene)-D-sorbitol (210 nm, 3.48 minutes);
1,3:2,4-di(4-methyl-benzylidene)-D-sorbitol (254 nm, 4.95 minutes); and
1,3:2,4-di(3,4-dimethyl-benzylidene)-D-sorbitol (254 nm, 6.26 minutes). Said retention time of the aromatic aldehydes varied and shifted slightly depending on different LC instruments and columns with different theoretical plate numbers.

### Example 1

### Preparation of pure 1,3:2,4-di(5-methyl-2-thenylidene)-D-sorbitol

AIL four-necked cylindrical shaped reaction flask equipped with a thermometer, a nitrogen inlet, and a mechanical stirrer was charged with D-sorbitol (20.0 g, 0.110 moles), methanesulfonic acid (1.00 g), 5-methyl-2-thiophenecarboxaldehyde (25.0 g, 0.198 moles) and methanol (200 ml) and then reacted at room temperature for 48 hours, so as to form a first reaction mixture.

Then potassium borohydride powders (1 g, purity>96%) were added in the first reaction mixture and stirred for 30 minutes to obtain a precipitated product. The precipitated product was filtered and washed with a 40 wt% methanol solution to obtain a second reaction mixture.

After that, the second reaction mixture was filtered to collect the solid substance therefrom, dried and ground to give 1,3:2,4-di(5-methyl-2-thenylidene)-D-sorbitol as fine white powders (29.5 g, yield 74.8%) having a melting point of 210.2°C to 210.9°C and a LC purity of 97.3%. No 5-methyl-2-thiophenecarboxaldehyde was detected by the GC analysis, and the fine white powders did not release any odor.

### Examples 2 to 6

### Preparation of pure 1,3:2,4-di(4-methylbenzylidene)-D-sorbitol

A 10 L four-necked cylindrical shaped reaction flask equipped with a thermometer, a nitrogen inlet, and a mechanical stirrer was charged with D-sorbitol (400 g, 2.20 moles), camphor sulfonic acid (12 g), 4-methyl-benzaldehyde (475 g, 3.95 moles) and methanol (4500 g) and then reacted at room temperature for 48 hours, so as to form a first reaction mixture.

Then the first reaction mixture was added with water (pH 8 to 10, 500 g) and stirred for 10 minutes, and added with the hydrogenating agent listed in Table 1 and stirred for 1 hour to obtain a precipitated product. The precipitated product was further washed with a 40 wt% methanol solution to obtain a second reaction mixture.

After filtration, a solid substance was collected from the second reaction mixture, dried and ground to give 1,3:2,4-di(4-methylbenzylidene)-D-sorbitol as white powders. Said white powders obtained from Examples 2 to 6 did not release any odor, and other characteristics thereof were listed in Table 1.

**Table 1: the usage of hydrogenating agent, the yield and LC purity of 1,3:2,4-di(4-methylbenzylidene)-D-sorbit of Examples 2 to 6, and their residue of 4-methylbenzaldehydes (impurities) determined by GC analyses.**

| Example No. | Hydrogenating agent | Usage of hydrogenating agent | Yield | Purity | Residue of 4-methylbenzaldehyde |
|---|---|---|---|---|---|
| 2 | VenPure™ SF (sodium borohydride powders, NaBH₄ effective amount>96%) | 0.5 g | 84.1% | 99.2% | 4 ppm |
| 3 | VenPure™ (sodium borohydride solution, 12% of NaBH₄ effective amount) | 5g | 82.6% | 99.8% | not detected |
| 4 | VenPure™ (sodium borohydride solution, 12% of NaBH₄ effective amount) | 10 g | 85.3% | 99.9% | not detected |
| 5 | VenPure™ (sodium borohydride solution, 20% of NaBH₄ effective amount, v/v 20/20) | 10 g | 85.1% | 99.5% | not detected |
| 6 | potassium borohydride, KBH₄ effective amount>96% | 5 g | 82.3% | 99.9% | not detected |

### Example 7

### Preparation of a composition comprising pure 1,3:2,4-di(3,4-dimethylbenzylidene)-D-sorbitol and organosilane treated fume silica powders

A 10 L four-necked cylindrical shaped reaction flask equipped with a thermometer, a nitrogen inlet, and a mechanical stirrer was charged with D-sorbitol (400 g, 2.20 moles), camphorsulfonic acid (12 g), 3,4-dimethyl-benzaldehyde (530 g, 3.95 moles) and methanol (4250 g), and then reacted at room temperature for 48 hours, so as to form a first reaction mixture.

After neutralizing the first reaction mixture to pH 8, VenPure™, 20 grams of sodium borohydride solution having 12% of NaBH₄ effective amount, and 10.6 grams of an organosilane treated fume silica, CAB-O-SIL® TS720, were added gently in the first reaction mixture and stirred for 2 hours to form a precipitated product. The precipitated product was washed with a 40 wt% methanol solution to obtain a second reaction mixture.

Finally, a solid substance was collected by filtration, dried and ground to give a composition (748.9 g) as white dispersive powders having 1,3:2,4-di(3,4-dimethylbenzylidene)-D-sorbitol and organosilane treated fume silica powders having a yield of 90.3%, an ash content of 1.3 % and a LC purity of 99.9%. No 3,4-dimethyl-benzaldehyde was detected by the GC analysis, and the composition did not release any odor.

### Comparative Example 1:

### Preparation of 1,3:2,4-di(5-methyl-2-thenylidene)-D-sorbitol

A 1 L four-necked cylindrical shaped reaction flask equipped with a thermometer, a nitrogen inlet, and a mechanical stirrer was charged with D-sorbitol (20.0 g, 0.110 moles), methanesulfonic acid (1.00 g), 5-methyl-2-thiophenecarboxaldehyde (25.0 g, 0.198 moles) and methanol (200 ml) to form a reaction mixture.

Then the reaction mixture was stirred at room temperature for 48 hours to form a precipitated product. A solid substance was further collected by filtration from the precipitated product, and washed with a 40 wt% methanol solution, dried and ground to give 1,3:2,4-di(5-methyl-2-thenylidene)-D-sorbitol as fine white powders (28.4 g, yield 72.1%) having a melting point of 209.4°C to 210.5°C and a LC purity of 96.3%. According to GC analysis, the residue of 5-methyl-2-thiophenecarboxaldehyde remaining in the final product was about 80 ppm. The fine white powders released odor.

### Comparative Example 2:

### Preparation of 1,3:2,4-di(4-methylbenzylidene)-D-sorbitol

A 10 L four-necked cylindrical shaped reaction flask equipped with a thermometer, a nitrogen inlet, and a mechanical stirrer was charged with D-sorbitol (400 g, 2.20 moles), camphor sulfonic acid (12 g), 4-methyl-benzaldehyde (475 g, 3.95 moles) and methanol (3800 g) to form a reaction mixture.

The reaction mixture was stirred at room temperature for 48 hours to form a precipitated product. A solid substance was further collected by filtration from the precipitated product, and washed with a 40 wt% methanol solution, dried and ground to give 1,3:2,4-di(4-methylbenzylidene)-D-sorbitol as fine white powders (641.7 g, yield 84.0%) having a melting point of 258.5°C to 263.2°C and a LC purity of 98%. According to GC analysis, the residue of 4-methyl-benzaldehyde remaining in the final product was about 140 ppm. The fine white powders released odor.

### Comparative Example 3:

### Preparation of a composition comprising

### 1,3:2,4-di(3,4-dimethylbenzylidene)-D-sorbitol and organosilane treated fume silica powders

A 10 L four-necked cylindrical shaped reaction flask equipped with a thermometer, a nitrogen inlet, and a mechanical stirrer was charged with D-sorbitol (400 g, 2.20 moles), camphorsulfonic acid (12 g), 3,4-dimethyl-benzaldehyde (530 g, 3.95 moles) and methanol (4250 g), and then reacted at room temperature for 48 hours, so as to form a reaction mixture.

After neutralizing the reaction mixture to pH 8 to 9 with a 4% sodium hydroxide solution, 15 grams of an organosilane treated fume silica, CAB-O-SIL® TS720, were added in the reaction mixture and stirred for 2 hours to form a precipitated product. The precipitated product was further washed with a 40 wt% methanol solution to obtain another reaction mixture.

Finally, a solid substance was collected from the reaction mixture by filtration, dried and ground to give a composition (739.9 g) comprising 1,3:2,4-di(3,4-dimethylbenzylidene)-D-sorbitol and organosilane treated fume silica powders as white dispersive powders having a yield of 88.5%, an ash content of 1.9 % and a LC purity of 99.2%. According to GC analysis, the residue of 3,4-dimethyl-benzaldehyde remaining in the final composition product was about 70 ppm.

### Test Example 1

### Color stability of pure 1,3:2,4-di(4-methylbenzylidene)-D-sorbitol at high temperature

In the instant test example, 50 grams of pure 1,3:2,4-di(4-methylbenzylidene)-D-sorbitol (Example 4) and commercial product (Millad® 3940) were independently thermally aged in a circulation oven for 2 hours to obtain the thermally treated samples. Then, the samples before and after thermal treatment were measured with HunterLab ColorFlex EZ color meter to determine their white indices (WIs) and yellow indices (YI). The higher WI represented the sample was closer to white, and the lower YI represented the sample did not appear yellow. The ΔWI was directed to the WI difference between the samples before and after thermal treatment, i.e., ΔWI=WI₁- WI₀; the ΔYI was directed to the YI difference between the samples before and after thermal treatment, i.e., ΔYI= YI₁-YI₀. The lower ΔWI and lower ΔYI represented that the sample had better color stability, and the results as shown in Table 2 were the averages determined by at least three experiments.

**Table 2: the color stabilities of Example 4 and commercial product (Millad® 3940)**

| Analysis Items | Example 4 | Millad® 3940 |
|---|---|---|
| WI before thermal treatment (WI₀) | 93.2 | 82.5 |
| WI after thermal treatment (WI₁) | 91.7 | 77.2 |
| WI difference (ΔWI) | 1.5 | 5.3 |
| YI before thermal treatment (YI₀) | -0.48 | 3.06 |
| YI after thermal treatment (YI₁) | 0.65 | 4.25 |
| YI difference (ΔYI) | 1.13 | 1.19 |

### Test Example 2

### Color stability of the composition comprising pure 1,3:2,4-di(3,4-dimethylbenzylidene)-D-sorbitol and organosilane treated fume silica powders at high temperature

In the instant test example, 50 grams of the composition comprising pure 1,3:2,4-di(3,4-dimethylbenzylidene)-D-sorbitol and organosilane treated fume silica powders (Example 7) and commercial product (Millad® 3988i) were independently thermally aged in a circulation oven for 2 hours to obtain the thermally treated samples. Said samples before and after thermal treatment were measured with the same manner and conditions as described in the test example 1, and the results were listed in Table 3.

**Table 3: the color stabilities of Example 7 and commercial product (Millad® 3988i)**

| Analysis Items | Example 7 | Millad® 3988i |
|---|---|---|
| WI before thermal treatment (WI₀) | 93.2 | 74.0 |
| WI after thermal treatment (WI₁) | 91.5 | 58.4 |
| WI difference (ΔWI) | 1.7 | 15.6 |
| YI before thermal treatment (YI₀) | -0.41 | 6.32 |
| YI after thermal treatment (YI₁) | 0.70 | 9.38 |
| YI difference (ΔYI) | 1.11 | 3.06 |

### Test Example 3

### Results of polypropylene composition

As shown in Table 4, the diacetal-based clarifying agents obtained by the aforementioned Examples, Comparative Examples and commercial products (Millad® 3940 and Millad® 3988i) were mixed with polypropylene, primary and secondary antioxidants and acid scavenger to form the polypropylene compositions as testing samples for evaluating the characteristics of the diacetal-based clarifying agent applied to the polypropylene polymer.

**Table 4 : the reagents and their usage for preparation of the polypropylene composition**

| Reagents | Usage |
|---|---|
| Polypropylene (ST611) | 100 phr |
| Primary Antioxidant (K-NOX 230) Cas No. 1709-70-2 | 0.06 phr |
| Secondary Antioxidant (K-NOX 168) Cas No. 31570-04-4 | 0.12 phr |
| Acid Scavenger (Calcium Stearate) | 0.08 phr |
| Diacetal-based clarifying agent | 0.22 phr |

The polypropylene polymer and all reagents weighed and blended. Each mixture was extruded under six heating zones at 180°C, 190°C, 215°C, 215°C, 215°C, and 190°C with a rotation of 40 revolutions per minute (rpm). Upon extruding the mixture out of the die, the odor released by the testing samples and a control sample were evaluated by the same five persons and rated as follows:
"0" represented that no odor difference between the odors released from the polypropylene composition and the control sample;
"1" represented that a very slight odor difference between the testing sample and the control sample;
"2" represented that a slight odor difference between the testing sample and the control sample;
"3" represented that a sensible odor difference between the testing sample and the control sample;
"4" represented that an obvious odor released by the testing sample during the process; and
"5" represented that a pungent odor released by the testing sample during the process.

The values determined by the five persons were averaged to give the "odor released at extrusion die", and the averages were listed in Table 5.

After that, the mixtures were loaded into an injection machine having three heating zones at 190°C, 230°C, and 230°C. The melting temperature of the outlet of the injection machine was set about 220°C to 230°C, the length/diameter ratio of the screw was about 24. Plaques of the testing samples and control sample were respectively obtained after the polypropylene compositions injected into a cooling mold. The plaques had a dimension of 142*108*2.54 mm³. During the process, the odor released by the polypropylene compositions of the testing samples and a control sample were also evaluated by the same five persons at the injection die, as "odor released at injection die" listed in Table 5. The odor released at injection die was also rated in the scale as follows:
"0" represented that no odor difference between the odors released from the polypropylene composition and the control sample;
"1" represented that a very slight odor difference between the testing sample and the control sample;
"2" represented that a slight odor difference between the testing sample and the control sample;
"3" represented that a sensible odor difference between the testing sample and the control sample;
"4" represented that an obvious odor released by the testing sample during the process; and
"5" represented that a pungent odor released by the testing sample during the process.

The haze values of the plaques of the testing samples and the control sample were measured with BYK Gardner XL-211 hazemeter according to ASTM Standard Test Method D1003-61, Standard Test Method for Haze and Luminous Transmittance of Transparent Plastics. The lower value represented that the plaques had higher transparency. The results were listed in Table 5.

After melting the polypropylene compositions under nitrogen and then cooling at 10°C/minute, the crystallization temperatures (Tc) of the testing samples and the control sample were determined with a differential scanning calorimeter (Mettler TOLEDO (DSC821e)), and the results were also listed in Table 5.

**Table 5: the crystallization temperature of the polypropylene compositions, the evaluations of the odor released at the extrusion die and the injection die, and the haze value of the plaques made from the polypropylene compositions of control sample, Examples 1, 2, 4, 5, and 7, Comparative Examples 1 to 3 and Commercial products (Millad® 3940 and Millad® 3988i)**

| Sample No. | Tc (DSC) | Evaluation of the odor released at extrusion die | Evaluation of odor released at injection die | Haze value |
|---|---|---|---|---|
| Control sample (without diacetal) | 107.1°C | 0 | 1 | 78.6 |
| Example 1 | 119.4°C | 1 | 2 | 29.5 |
| Example 2 | 119.7°C | 1 | 2 | 28.6 |
| Example 4 | 120.6°C | 0 | 1 | 28.1 |
| Example 5 | 119.9°C | 1 | 1 | 27.6 |
| Example 7 | 122.9°C | 0 | 1 | 24.6 |
| Comparative Example 1 | 119.0°C | 3 | 4 | 30.1 |
| Comparative Example 2 | 120.6°C | 2 | 4 | 28.8 |
| Comparative Example 3 | 123.7°C | 1 | 2 | 25.0 |
| Commercial Product (Millad® 3940) | 119.7°C | 2 | 4 | 28.6 |
| Commercial Product (Millad® 3988i) | 122.8°C | 1 | 2 | 25.5 |

Based on the foregoing results, the diacetal-based clarifying agent prepared by the method is highly pure and substantially free of impurities and volatile substances. Such a diacetal-based clarifying agent does not release intolerable odor during the plastic processing, and prevents the final plastic product from yellowing at a temperature higher than 190°C. Accordingly, the diacetal-based clarifying agent prepared by the method can guarantee the final plastic product's safety in use and allow good color and thermal stabilities of the final plastic product, thereby being applicable to widely used in various applications.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A method of preparing a diacetal-based clarifying agent, **characterized in** comprising:
(a) mixing an aromatic aldehyde, a polyol, and an acid catalyst in a polar organic solvent to obtain a first reaction mixture, wherein an equivalence ratio of said aromatic aldehyde to said polyol ranges from 2:1 to 2:2;
(b) adding a hydrogenating agent into the first reaction mixture to obtain a precipitated product, wherein an equivalence ratio of the hydrogenating agent to said aromatic aldehyde is more than 0.01:1;
(c) mixing methanol and the precipitated product to obtain a second reaction mixture;
(d) filtering the second reaction mixture to obtain a solid substance; and
(e) drying and grinding the solid substance to obtain the diacetal-based clarifying agent;
wherein the diacetal-based clarifying agent is a compound selected from the group consisting of compounds represented by formulae (I) to (V): 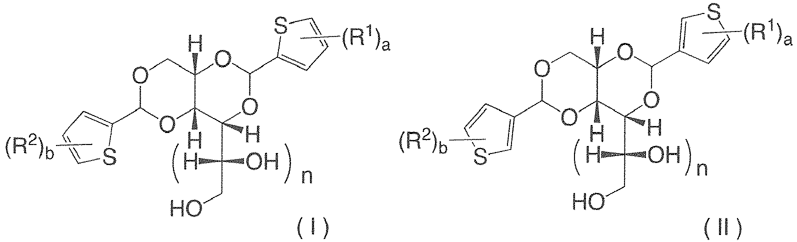 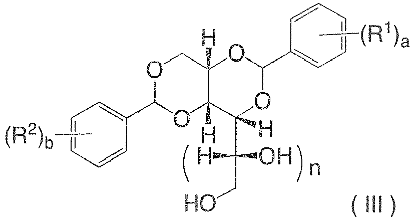 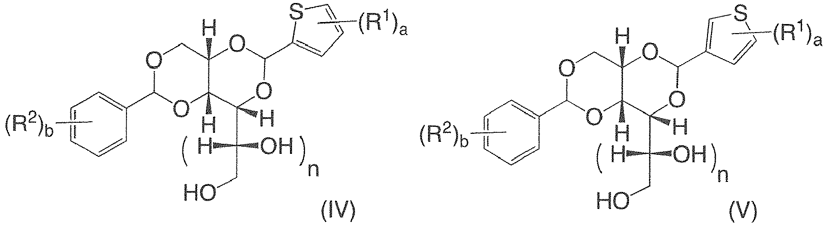 wherein R¹ and R² are each independently selected from the group consisting of a hydrogen group, an alkyl group having 1 to 4 carbon atoms, an alkoxy group having 1 to 4 carbon atoms, an alkoxycarbonyl group having 1 to 4 carbon atoms, a fluoro group, a chloro group, and a bromo group; wherein a and b are each independently an integral from 0 to 3; and wherein n is 0 or 1.

2. The method as claimed in claim 1, **characterized in that** the polar organic solvent is selected from the group consisting of methanol, ethanol, dimethyl formamide, acetonitrile, water, and combinations thereof.

3. The method as claimed in claim 1, **characterized in that** said aromatic aldehyde is a thiophenecarboxaldehyde-based compound, a benzaldehyde-based compound or their combination.

4. The method as claimed in claim 3, **characterized in that** said thiophenecarboxaldehyde-based compound is 3-thiophenecarboxaldehyde or 5-methyl-2-thiophenecarboxaldehyde.

5. The method as claimed in claim 3, **characterized in that** said benzaldehyde-based compound is 4-methyl-benzaldehyde, 4-ethyl-benzaldehyde, or 3,4-dimethyl-benzaldehyde.

6. The method as claimed in claim 1, **characterized in that** said polyol is sorbitol or xylitol.

7. The method as claimed in claim 1, **characterized in that** the acid catalyst is sulfuric acid, phosphoric acid, hydrochloric acid, methanesulfonic acid, camphor sulfonic acid, *p*-toluene sulfonic acid or naphthalene sulfonic acid.

8. The method as claimed in claim 1, **characterized in that** the equivalence ratio of said aromatic aldehyde to said polyol ranges from 2:1.05 to 2:1.3.

9. The method as claimed in claim 1, **characterized in that** the hydrogenating agent is selected from the group consisting of: sodium hydride, potassium hydride, aluminium hydride, sodium cyanoborohydride, diisobutylaluminium hydride, lithium borohydride, sodium borohydride, potassium borohydride, calcium borohydride, and combinations thereof.

10. The method as claimed in claim 9, **characterized in that** the hydrogenating agent is sodium borohydride or potassium borohydride.

11. The method as claimed in claim 1, **characterized in that** the equivalence ratio of the hydrogenating agent to said aromatic aldehyde is more than 0.03:1 to 0.3: 1.

12. The method as claimed in claim 1, **characterized in that** the step (b) comprises:
(b1) adding the hydrogenating agent into the first reaction mixture; and
(b2) adjusting the pH value of the first reaction mixture within a range of pH 7 to pH 13, so as to obtain the precipitated product.

## Patentansprüche

1. Ein Verfahren zur Herstellung eines diacetalbasierten Klärmittels, **dadurch gekennzeichnet, dass** es umfasst:
(a) Mischen eines aromatischen Aldehyds, eines Polyols und eines Säurekatalysators in einem polaren organischen Lösungsmittel, um eine erste Reaktionsmischung zu erhalten, wobei ein Äquivalentverhältnis des aromatischen Aldehyds zu dem Polyol von 2:1 bis 2:2 reicht;
(b) Zugeben eines Hydrierungsmittels in die erste Reaktionsmischung, um ein gefälltes Produkt zu erhalten, wobei ein Äquivalentverhältnis des Hydrierungsmittels zu dem aromatischen Aldehyd größer als 0,01:1 ist;
(c) Mischen von Methanol mit dem gefällten Produkt, um eine zweite Reaktionsmischung zu erhalten;
(d) Filtrieren der zweiten Reaktionsmischung, um einen Feststoff zu erhalten; und
(e) Trocknen und Zermahlen des Feststoffes, um das auf diacetalbasierende Klärmittel zu erhalten;
wobei das diacetalbasierende Klärmittel eine Verbindung ist, ausgewählt aus der Gruppe bestehend aus Verbindungen, die durch die Formen (I) bis (V) dargestellt sind: 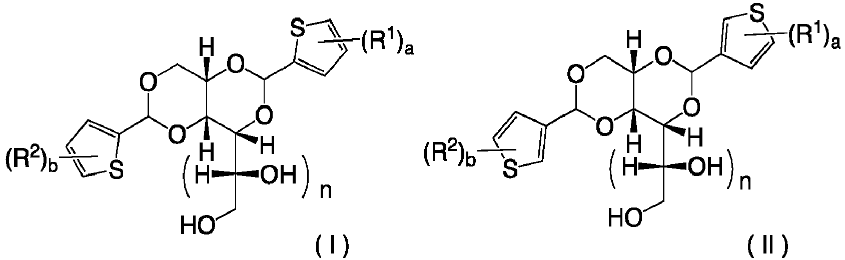 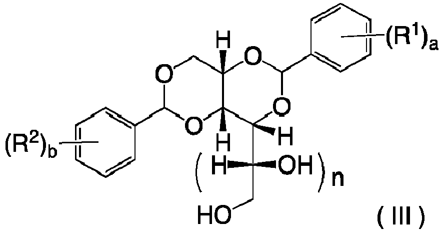 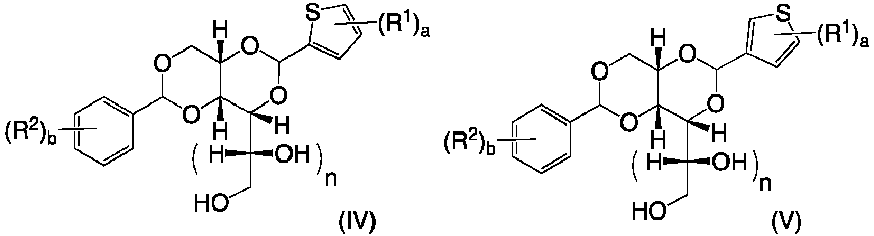 worin R¹ und R² jeweils unabhängig voneinander ausgewählt sind aus der Gruppe bestehend aus einer Wasserstoffgruppe, einer Alkylgruppe mit 1 bis 4 Kohlenstoffatomen, einer Alkoxygruppe mit 1 bis 4 Kohlenstoffatomen, einer Alkoxycarbonylgruppe mit 1 bis 4 Kohlenstoffatomen, einer Fluorgruppe, einer Chlorgruppe und einer Bromgruppe, wobei a und b jeweils unabhängig voneinander eine ganze Zahl von 0 bis 3 sind und wobei n gleich 0 oder 1 ist.

2. Das Verfahren gemäß Anspruch 1, **dadurch gekennzeichnet, dass** das polare organische Lösungsmittel ausgewählt ist aus der Gruppe bestehend aus Methanol, Ethanol, Dimethylformamid, Acetonitril, Wasser und Kombinationen daraus.

3. Das Verfahren gemäß Anspruch 1, **dadurch gekennzeichnet, dass** das aromatische Aldehyd eine Verbindung auf Basis eines Thiophencarboxaldehyds, eine Verbindung auf Basis eines Benzaldehyds oder deren Kombination ist.

4. Das Verfahren gemäß Anspruch 3, **dadurch gekennzeichnet, dass** die Verbindung auf Thiophencarboxaldehydbasis 3-Thiophencarboxaldehyd oder 5-Methyl-2-thiophencarboxaldehyd ist.

5. Das Verfahren gemäß Anspruch 3, dadurch charakterisiert, dass die Verbindung auf Benzaldehydbasis 4-Methylbenzaldehyd, 4-Ethylbenzaldehyd oder 3,4-Dimethylbenzaldehyd ist.

6. Das Verfahren gemäß Anspruch 1, **dadurch gekennzeichnet, dass** das Polyol Sorbit oder Xylit ist.

7. Das Verfahren gemäß Anspruch 1, dadurch charakterisiert, dass der Säurekatalysator Schwefelsäure, Phosphorsäure, Salzsäure, Methansulfonsäure, Camphersulfonsäure, *p*-Toluolsulfonsäure oder Naphtalinsulfonsäure ist.

8. Das Verfahren gemäß Anspruch 1, **dadurch gekennzeichnet, dass** das Äquivalentverhältnis des aromatischen Aldehyds zu dem Polyol von 2:1,05 bis 2:1,3 reicht.

9. Das Verfahren gemäß Anspruch 1, **dadurch gekennzeichnet, dass** das Hydrierungsmittel ausgewählt ist aus der Gruppe bestehend aus Natriumhydrid, Kaliumhydrid, Aluminiumhydrid, Natriumcyanoborhydrid, Diisobutylaluminiumhydrid, Lithiumborhydrid, Natriumborhydrid, Kaliumborhydrid, Calciumborhydrid und Kombinationen daraus.

10. Das Verfahren gemäß Anspruch 9, dadurch gekennzeichent, dass das Hydrierungsmittel Natriumborhydrid oder Kaliumborhydrid ist.

11. Das Verfahren gemäß Anspruch 1, **dadurch gekennzeichnet, dass** das Äquivalentverhältnis des Hydrierungsmittels zu dem aromatischen Aldehyd größer als 0,03:1 bis 0,3:1 ist.

12. Das Verfahren gemäß Anspruch 1, **dadurch gekennzeichnet, dass** der Schritt (b) umfasst:
(b1) die Zugabe des Hydrierungsmittels in die erste Reaktionsmischung und
(b2) das Einstellen des pH-Wertes der ersten Reaktionsmischung innerhalb eines Bereichs von pH 7 bis pH 13, um das gefällte Produkt zu erhalten.

## Revendications

1. Procédé de préparation d'un agent de clarification à base de diacétal, **caractérisé en ce qu'**il comprend :
(a) le mélange d'un aldéhyde aromatique, d'un polyol, et d'un catalyseur acide dans un solvant organique polaire pour obtenir un premier mélange de réaction, dans lequel un rapport d'équivalence entre ledit aldéhyde aromatique et ledit polyol va de 2:1 à 2:2 ;
(b) l'ajout d'un agent d'hydrogénation dans le premier mélange de réaction pour obtenir un produit précipité, dans lequel un rapport d'équivalence entre l'agent d'hydrogénation et ledit aldéhyde aromatique est supérieur à 0,01:1 ;
(c) le mélange de méthanol et du produit précipité pour obtenir un second mélange de réaction ;
(d) la filtration du second mélange de réaction pour obtenir une substance solide ; et
(e) le séchage et le broyage de la substance solide pour obtenir l'agent de clarification à base de diacétal ;
dans lequel l'agent de clarification à base de diacétal est un composé choisi dans le groupe consistant en des composés représentés par les formules (I) à (V) : 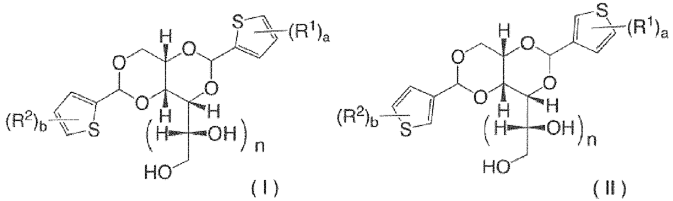 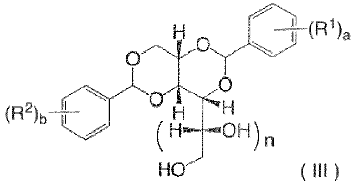 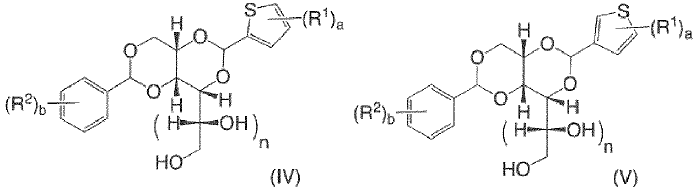 dans lequel R¹ et R² sont chacun indépendamment choisis dans le groupe consistant en un groupe hydrogène, un groupe alkyle ayant de 1 à 4 atomes de carbone, un groupe alcoxy ayant de 1 à 4 atomes de carbone, un groupe alcoxycarbonyle ayant de 1 à 4 atomes de carbone, un groupe fluoro, un groupe chloro, et un groupe bromo ; dans lequel a et b sont chacun indépendamment un entier de 0 à 3 ; et dans lequel n vaut 0 ou 1.

2. Procédé selon la revendication 1, **caractérisé en ce que** le solvant organique polaire est choisi dans le groupe consistant en le méthanol, l'éthanol, le diméthylformamide, l'acétonitrile, l'eau, et leurs combinaisons.

3. Procédé selon la revendication 1, **caractérisé en ce que** ledit aldéhyde aromatique est un composé à base de thiophènecarboxaldéhyde, un composé à base de benzaldéhyde ou leur combinaison.

4. Procédé selon la revendication 3, **caractérisé en ce que** ledit composé à base de thiophènecarboxaldéhyde est le 3-thiophènecarboxaldéhyde ou le 5-méthyl-2-thiophènecarboxaldéhyde.

5. Procédé selon la revendication 3, **caractérisé en ce que** ledit composé à base de benzaldéhyde est le 4-méthyl-benzaldéhyde, le 4-éthyl-benzaldéhyde, ou le 3,4-diméthyl-benzaldéhyde.

6. Procédé selon la revendication 1, **caractérisé en ce que** ledit polyol est le sorbitol ou le xylitol.

7. Procédé selon la revendication 1, **caractérisé en ce que** le catalyseur acide est l'acide sulfurique, l'acide phosphorique, l'acide chlorhydrique, l'acide méthanesulfonique, l'acide camphre sulfonique, l'acide *p*-toluène sulfonique ou l'acide naphtalène sulfonique.

8. Procédé selon la revendication 1, **caractérisé en ce que** le rapport d'équivalence entre ledit aldéhyde aromatique et ledit polyol va de 2:1,05 à 2:1,3.

9. Procédé selon la revendication 1, **caractérisé en ce que** l'agent d'hydrogénation est choisi dans le groupe consistant en : l'hydrure de sodium, l'hydrure de potassium, l'hydrure d'aluminium, le cyanoborohydrure de sodium, l'hydrure de diisobutylaluminium, le borohydrure de lithium, le borohydrure de sodium, le borohydrure de potassium, le borohydrure de calcium, et leurs combinaisons.

10. Procédé selon la revendication 9, **caractérisé en ce que** l'agent d'hydrogénation est le borohydrure de sodium ou le borohydrure de potassium.

11. Procédé selon la revendication 1, **caractérisé en ce que** le rapport d'équivalence entre l'agent d'hydrogénation et ledit aldéhyde aromatique est supérieur à 0,03:1 à 0,3:1.

12. Procédé selon la revendication 1, **caractérisé en ce que** l'étape (b) comprend :
(b1) l'ajout de l'agent d'hydrogénation dans le premier mélange de réaction ; et
(b2) l'ajustement de la valeur de pH du premier mélange de réaction dans une plage de pH 7 à pH 13, de façon à obtenir le produit précipité.
